# NOUVEAU FASCICULE DE BREVET EUROPEEN

(11) **EP 3 554 458 B2**
(45) Date de publication et mention de la décision concernant l'opposition: **29.07.2026**
(45) Mention de la délivrance du brevet: 28.06.2023
(21) Numéro de dépôt: 17822399.6
(22) Date de dépôt: 12.12.2017
(51) Int. Cl.: A61K 8/06, A61K 8/34, A61K 8/31, A61Q 19/00

(54) **UTILISATION D'UN MÉLANGE D'HYDROCARBURES POUR AMÉLIORER LES PROPRIÉTÉS SENSORIELLES D'ÉMULSIONS HUILE-DANS-EAU À BASE DE GLYCÉRINE**
VERWENDUNG EINER KOHLENWASSERSTOFF-MISCHUNG ZUR VERBESSERUNG DER SENSORISCHEN EIGENSCHAFTEN VON GLYCERIN ENTHALTENDEN ÖL-IN-WASSER-EMULSIONEN
USE OF A HYDROCARBON MIXTURE TO IMPROVE THE SENSORIAL PROPERTIES OF GLYCERIN-BASED OIL-IN-WATER EMULSIONS

(30) Priorité: 16.12.2016 FR 1662647
(43) Date de publication de la demande: 23.10.2019
(73) Titulaire: Société d'Exploitation de Produits pour les Industries Chimiques SEPPIC, 75007 Paris (FR); TotalEnergies OneTech, 92400 Courbevoie (FR)
(72) Inventeur: CAMBOS, Sophie, 81105 Castres (FR); MERAT, Emmanuelle, 81440 Lautrec (FR); TAILLEBOIS, Cécile, 81990 Salies (FR); SWOBODA, Benjamin, 78630 Orgeval (FR)
(74) Mandataire: Alatis
(86) Numéro de dépôt international: PCT/FR2017/053507
(87) Numéro de publication internationale: WO 2018/109353

(56) Documents cités:
- EP-A1- 1 889 596
- DE-A1- 102015 204 662
- JP-A- 2007 145 719
- US-A1- 2006 167 117
- US-A1- 2013 150 322
- US-A1- 2015 125 403
- AGRANA/AAK SWEDEN AB: "Winter Comfort Eco-conscious Hand Cream MAISITA 9040", INTERNET CITATION, 24 October 2016 (2016-10-24), XP002770404, Retrieved from the Internet <URL:http://www.agrana.com/de/downloadcenter/?layout=thumbs&id=3739&no_cache=1&itemsPerPage=36> [retrieved on 20170516]
- "Atomizing sprays for sun care applications", IP.COM JOURNAL, IP.COM INC., WEST HENRIETTA, NY, US, 30 November 2006 (2006-11-30), XP013116940, ISSN: 1533-0001
- DATABASE GNPD [online] MINTEL; November 2016 (2016-11-01), ANONYMOUS: "Smooth Milk Body Lotion", XP002772790, retrieved from http://www.gnpd.com Database accession no. 4392919

## Description

L'invention a pour objet une nouvelle utilisation pour améliorer les propriétés sensorielles d'émulsions huile-dans-eau comportant de la glycérine et réduire leur effet collant.

Il est bien connu d'épaissir des phases aqueuses destinées à des applications cosmétiques, dermopharmaceutiques ou pharmaceutiques, pour préparer des émulsions de type huile-dans-eau, en y introduisant des polymères hydrophiles synthétiques ou naturels. Les polymères naturels tels que les gommes de xanthane ou de guar sont assez largement utilisés mais présentent les inconvénients classiques des produits naturels (qualité et prix fluctuants).

C'est pourquoi, les polymères épaississants synthétiques sont largement utilisés dans les industrie cosmétique ou pharmaceutique. Des épaississants fonctionnant sur une large gamme de pH et ayant l'avantage d'être particulièrement bien tolérés, ont été déjà proposés par plusieurs compagnies dont la demanderesse. On peut notamment citer les épaississants synthétiques décrits dans les brevets US 6,197,287, US 6,136,305, US 6,346,239 ou encore EP 0 503,853, ou encore US 5,004,598. Ces polymères se présentent sous forme de latex inverse ou de poudre. Ils sont essentiellement destinés à épaissir des phases aqueuses contenant les différents constituants classiques que l'on peut trouver dans des formulations topiques de l'industrie cosmétique ou pharmaceutique. On citera notamment des huiles, des tensioactifs (non ioniques ou anioniques) encore appelés émulsionnants, des sels minéraux, des acides faibles.

Or certaines formulations, plus particulièrement celles destinées au soin de la peau, contiennent aussi des quantités relativement importantes de glycérine (ou glycérol), typiquement entre 5% et 10% massique, pour augmenter leur potentiel hydratant. Mais comme la présence de glycérine en leur sein augmente aussi considérablement leur effet collant, les préparateurs y ajoutent des huiles de silicone pour limiter ou annihiler cet effet collant.

Cependant l'addition d'huiles de silicone complexifie la préparation de ces formules. De plus, la présence d'huiles de silicone dans des formules, qui sont destinées à être en contact direct avec la peau, est mal perçue par le consommateur final. L'industrie cosmétique essaye donc d'en limiter l'utilisation, et la demanderesse a notamment mis au point de nouveaux polymères synthétiques épaississants qui ont été divulgués dans les demandes internationales publiées respectivement sous les numéros WO2013132169 A1 et WO2013128095 A1.

Cependant, ces structures polymériques impliquent toujours l'utilisation de monomères portant soit des groupements fonctionnels siliconés soit des groupements fonctionnels fluorés, dont les industries des cosmétiques cherchent à ne plus utiliser pour des raisons environnementales et réglementaires.

Les inventeurs ont donc cherché à développer une nouvelle solution pour améliorer les propriétés sensorielles d'une formulation, dans le but de réduire ou d'annihiler l'effet collant induit par la présence de glycérine sans qu'il soit nécessaire d'épaissir la phase aqueuse avec des polymères synthétiques comportant des motifs siliconés ou fluorés, ni d'ajouter un tiers composé de nature siliconée.

C'est pourquoi selon un premier aspect, l'invention a pour objet l'utilisation d'une quantité efficace d'un mélange (M₁) d'hydrocarbures saturés cycliques ou acycliques, linéaires ou ramifiés parmi lesquels au moins 95% massique comportent de quinze à dix-neuf atomes de carbone pour améliorer les propriétés sensorielles d'une émulsion à usage topique (E₀) de type huile-dans-eau comprenant pour 100% de sa masse, de 1% à 30% massique de glycérol, plus particulièrement de 5% à 30%, et encore plus particulièrement de 5% à 25% massique de glycérol, dans le but de réduire ou d'annihiler l'effet collant induit par la présence de glycérine sans qu'il soit nécessaire d'épaissir la phase aqueuse avec des polymères synthétiques comportant des motifs siliconés ou fluorés, ni d'ajouter un tiers composé de nature siliconée.

Par quantité efficace, on désigne, dans la définition du procédé tel que défini ci-dessus, une quantité telle, que l'émulsion à usage topique (E) de type huile-dans-eau résultant dudit procédé ci-dessus :
- Montre une valeur moyenne, de trois mesures de la force d'adhésion, inférieure ou égale à 9N, et plus particulièrement inférieure ou égale à 3N, ladite force d'adhésion étant enregistrée lors de chacune des mesures par l'intermédiaire d'un rhéomètre DHR2 (TA Instrument) équipé d'un support de type plan Peltier dont la température est régulée à 40°C, sur lequel est posé une surface de plexiglas sur laquelle est déposée l'émulsion à tester et
- Montre un aspect homogène après stockage d'une durée d'un mois à une température de 45°C.

Par émulsion à usage topique (E₀) topique de type huile-dans-eau, on désigne au sens de la présente invention les émulsions comprenant pour 100% massique :
- de 95% à 50%, plus particulièrement de 90% à 70% d'une phase aqueuse (A₀) cosmétiquement acceptable,
- de 5% à 50%, plus particulièrement de 10% à 30% d'une phase grasse (Go), ladite phase grasse (Go) comprenant pour 100% de sa masse, de 1% à 12%, plus particulièrement de 2% à 8% d'au moins un agent tensioactif de type huile-dans-eau et de 88% à 99%, plus particulièrement de 92% à 98% d'au moins une huile et/ou une cire.

Par « huile » présente dans la phase grasse (Go) de l'émulsion à usage topique (E₀) topique de type huile-dans-eau telle que définie précédemment, on désigne au sens de la présente invention les substances chimiques ou les mélanges de substances chimiques insolubles dans l'eau, et se présentant sous un aspect liquide à une température de 25°C.

Par « cire » présente dans la phase grasse (Go) de l'émulsion à usage topique (E₀) topique de type huile-dans-eau telle que définie précédemment, on désigne au sens de la présente invention les substances chimiques ou les mélanges de substances chimiques insolubles dans l'eau, et se présentant sous un aspect solide à une température de 45°C.

Par «agent tensioactif de type huile-dans-eau » présent dans la phase grasse (Go) de l'émulsion à usage topique (E₀) topique de type huile-dans-eau telle que définie précédemment, on désigne au sens de la présente invention, la substance chimique ou le mélange de substances chimiques qui permet de stabiliser les gouttelettes de ladite phase grasse (Go) en dispersion dans la phase continue (A₀).

Comme agent tensioactif de type huile-dans-eau présent dans la phase grasse (Go) de l'émulsion à usage topique (E₀) topique de type huile-dans-eau telle que définie précédemment, il y a par exemple :
- Des polysorbates résultant de la réaction d'éthoxylation entre un équivalent molaire d'esters de sorbitan et entre 5 et 20 équivalents molaires d'oxyde d'éthylène, et plus particulièrement entre un équivalent molaire de laurate de sorbitan, ou de palmitate de sorbitan, ou de stéarate de sorbitan, ou d'isostéarate de sorbitan, ou d'oléate de sorbitan, et entre 5 et 20 équivalents molaires d'oxyde d'éthylène ;
- Les produits résultant de la réaction d'éthoxylation entre un équivalent molaire d'un acide gras, comme par exemple l'acide palmitique, l'acide myristique, l'acide laurique, l'acide stéarique, l'acide isostéarique, l'acide oléique, et entre 5 à 40 équivalents molaires d'oxyde d'éthylène ;
- Les produits résultant de la réaction d'estérification entre un acide gras, comme par exemple l'acide palmitique, l'acide myristique, l'acide laurique, l'acide stéarique, l'acide isostéarique, l'acide oléique, l'acide arachidique, l'acide béhénique et entre 4 à 20 équivalents molaires, plus particulièrement entre 3 à 10 équivalents molaires de glycérol.

L'expression "cosmétiquement acceptable" utilisée dans la définition de la phase aqueuse (A₀) de l'émulsion à usage topique de type huile-dans-eau, signifie, selon la directive du Conseil de la Communauté Economique Européenne N°76/768/CEE du 27 juillet 1976 modifiée par la directive N°93/35/CEE du 14 juin 1993, toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état. Un milieu cosmétiquement acceptable de ces compositions objet de l'invention peut contenir classiquement de l'eau, un ou plusieurs solvants organiques cosmétiquement acceptables, un mélange d'eau et d'un ou plusieurs solvants organiques. Les solvants cosmétiquement acceptables peuvent plus particulièrement être choisis parmi les alcools polyhydriques comme par exemple le glycérol, le diglycérol, les oligomères du glycérol, l'éthylène glycol, le propylène glycol, l'hexylène glycol, le diéthylène glycol, le xylitol, l'érythritol, le sorbitol, ou les alcools hydrosolubles.

Selon un mode particulier de l'utilisation revendiquée, par quantité efficace dudit mélange (M₁), on désigne une proportion massique de 1% à 25% de l'émulsion huile-dans-eau, tout particulièrement de 5% à 20%.

L'expression "à usage topique" utilisée dans la définition du procédé tel que défini ci-dessus, signifie que ladite composition est mise en oeuvre par application sur la peau, les cheveux, le cuir chevelu ou les muqueuses, qu'il s'agisse d'une application directe dans le cas d'une composition cosmétique, dermocosmétique, dermopharmaceutique ou pharmaceutique ou d'une application indirecte par exemple dans le cas d'un produit d'hygiène corporelle sous forme de lingette en textile ou en papier ou de produits sanitaires destinés à être en contact avec la peau ou les muqueuses.

Par « alcanes linéaires » présents dans le mélange (M₁) mis en oeuvre dans le procédé objet de la présente invention, et comportant de quinze à dix-neuf atomes de carbone, on désigne plus particulièrement au sens de la présente invention les éléments choisis parmi le groupe constitué par le pentadécane, l'hexadécane, l'heptadécane, l'octadécane et le nonadécane.

Par « alcanes ramifiés » présents dans le mélange (M₁) mis en oeuvre dans le procédé objet de la présente invention, et comportant de quinze à dix-neuf atomes de carbone, on désigne plus particulièrement au sens de la présente invention les éléments choisis parmi le groupe constitué par le 2-méthyl tétradécane (ou isopentadécane), le 2-méthyl pentadécane (ou isohexadécane), le 2-méthyl hexadécane (ou isoheptadécane), le 2-méthyl heptadécane (ou isooctadécane) et le 2-méthyl octadécane (ou isononadécane).

Par cyclo-alcanes présents dans le mélange (M₁) mis en oeuvre dans le procédé objet de la présente invention, et comportant de 15 à 19 atomes de carbone, on désigne plus particulièrement au sens de la présente invention des hydrocarbures saturés comprenant au moins un groupe hydrocarboné cyclique saturé optionnellement substitué par un ou plusieurs radicaux alkyles linéaires ou ramifies.

Selon un aspect particulier, ledit mélange (M₁) comprend pour 100% de sa masse :
- Une proportion massique en alcanes ramifiés supérieure ou égale à 80 % et inférieure ou égale à 100%, et plus particulièrement supérieure ou égale à 90% massique ;
- Une proportion massique en alcanes linéaires supérieure ou égale à 0% et inférieure ou égale à 15%, et plus particulièrement inférieure ou égale à 10% massique,
- Une proportion massique en cyclo-alcanes supérieure ou égale à 0% et inférieure ou égale à 5%, et plus particulièrement inférieure ou égale à 1% massique, et en ce que de 95% massique à 100% massique desdits hydrocarbures cycliques ou acycliques, linéaires ou ramifiés, comportent de quinze à dix-neuf atomes de carbones et en ce que jusqu'à 5% massique desdits hydrocarbures cycliques, acycliques, linéaires ou ramifiés comportent moins de quinze atomes de carbone ou plus de dix-neuf atomes de carbone.

Selon un aspect plus particulier, le mélange (M₁) est un mélange d'hydrocarbures saturés commercialisé sous le nom de marque Emogreen^{™}L15 comprenant pour 100% de sa masse
i) 3,7% d'alcanes linéaires comportant de quinze à dix-neuf atomes de carbone,
ii) 96% d'iso-alcanes comportant de quinze à dix-neuf atomes de carbone, et
iii) 0,3% de cyclo-alcanes comportant de quinze à dix-neuf atomes de carbone.

Selon un autre aspect plus particulier, ledit mélange (M₁) comprend pour 100% de sa masse :
- Une proportion massique en alcanes ramifiés supérieure ou égale à 40% et inférieure ou égale à 100%, et plus particulièrement supérieure ou égale à 50% massique,
- Une proportion massique en alcanes linéaires supérieure ou égale à 0% et inférieure ou égale à 20%, et plus particulièrement inférieure ou égale à 15% massique,
- Une proportion massique en cyclo-alcanes supérieure ou égale à 0% et inférieure ou égale à 40% et plus particulièrement inférieure ou égale à 35% massique,
et en ce que 100% massique desdits hydrocarbures cycliques ou acycliques, linéaires ou ramifiés, comportent de quinze à dix-neuf atomes de carbones.

Selon un aspect tout particulier, le mélange (M₁) est un mélange d'hydrocarbures saturés commercialisé sous le nom de marque Emosmart^{™}L19, comprenant pour 100% de sa masse
i) 13,20% massique d'alcanes linéaires comportant de 15 à 19 atomes de carbone,
ii) 55,00% massique d'iso-alcanes comportant de 15 à 19 atomes de carbone, et
iii) 31,80% de cyclo-alcanes comportant de 15 à 19 atomes de carbone.

Selon un autre aspect particulier de l'utilisation revendiquée, la quantité efficace dudit mélange (M₁) est telle que le ratio massique entre le glycérol et ledit mélange (M₁) est inférieur ou égal à 10 et supérieur ou égal à 1, et plus particulièrement inférieur ou égal à 5 et supérieur ou égal à 2.

L' émulsion à usage topique de type huile-dans-eau (E) dont il est question peut comprendre pour 100% de sa masse :
- de 40% à 90%, massique, plus particulièrement de 60% à 90% massique, et encore plus particulièrement de 70% à 90% massique d'une phase aqueuse (A) cosmétiquement acceptable comprenant pour 100% de sa masse de 5% à 30%, et encore plus particulièrement de 5% à 25% massique de glycérol, et
- de 10% à 60%, plus particulièrement de 10% à 40%, et encore plus particulièrement de 10% à 30% massique d'une phase grasse (G) comprenant pour 100% de sa masse de 1% à 25% massique, tout particulièrement de 5% à 20%, dudit mélange (M₁) d'hydrocarbures saturés cycliques ou acycliques, linéaires ou ramifiés parmi lesquels au moins 95% massique comporte de quinze à dix-neuf atomes de carbone et de 0,5% à 15 % d'au moins un agent tensioactif de type huile-dans-eau.

Par "phase grasse (G)", on désigne au sens de la présente invention, un corps gras ou un mélange de corps gras insoluble dans l'eau et/ou dans les mélanges d'eau et de solvants polaires. Une telle « phase grasse » peut comprendre des huiles et/ou des cires. Parmi les éléments constitutifs de la phase grasse, on peut citer :
- Les huiles d'origine animale, telles que le squalène ou le squalane ;
- les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de mais, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, l'huile de sésame, l'huile de reine des prés, l'huile de Macadamia kiwi, l'huile de bourrache, l'huile de pépins de cassis, l'huile de café, l'huile de pistache, l'huile de noyau de pêche, l'huile de pépin de framboise, l'huile de pépin de fraise, l'huile de melon, l'huile de pépin de myrtille, l'huile d'argan, l'extrait huileux de prune, l'huile de grenade, l'huile de papaye, l'huile de lait de coco, les huiles issues de fleurs ou de légumes;
- Les huiles végétales éthoxylées ;
- Les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les polyalphaoléfines, les polyoléfines comme le polyisobutène, le polydécène hydrogéné ou le polyisobutène hydrogéné, commercialisé en France par la société Ets B. Rossow et Cie sous le nom PARLEAM - POLYSYNLANE^{™}, cité dans: Michel and Irene Ash; Thesaurus of Chemical Products, Chemical Publishing Co, Inc. 1986 Volume I, page 211 (ISBN 0 7131 3603 0);
- Les huiles de silicone comme les diméthylpolysiloxanes, les méthylphényl-polysiloxanes, les silicones modifiées par des amines, les silicones modifiées par des acides gras, les silicones modifiées par des alcools, les silicones modifiées par des alcools et des acides gras, des silicones modifiées par des groupements polyéther, des silicones époxy modifiées, des silicones modifiées par des groupements fluorés, des silicones cycliques et des silicones modifiées par des groupements alkyles.
- Des cires comme la cire d'abeille, la cire de carnauba, la cire de candelilla, la cire d'ouricoury, la cire du Japon, la cire de fibre de liège, la cire de canne à sucre, la cire de jojoba, la cire de fleurs de cassis, la cire de fleurs de narcisse, la cire de fleurs d'oranger, la cire d'orange, la cire de riz, les cires de paraffines, les cires de lignite, les cires microcristallines, la cire de lanoline; l'ozokérite; la cire de polyéthylène, les cires de silicone; les cires végétales; les alcools gras et les acides gras solides à température ambiante; les glycérides solides à température ambiante.

Par «agent tensioactif de type huile-dans-eau » présent dans la phase grasse (G) de l'émulsion à usage topique (E) de type huile-dans-eau telle que définie précédemment, une substance chimique ou le mélange de substance chimique qui permet de stabiliser les gouttelettes de la phase grasse (G) en dispersion dans la phase continue (A). On peut citer par exemple :
- Des polysorbates résultant de la réaction d'éthoxylation entre un équivalent molaire d'esters de sorbitan et entre 5 et 20 équivalents molaires d'oxyde d'éthylène, et plus particulièrement entre un équivalent molaire de laurate de sorbitan, ou de palmitate de sorbitan, ou de stéarate de sorbitan, ou d'isostéarate de sorbitan, ou d'oléate de sorbitan, et entre 5 et 20 équivalents molaires d'oxyde d'éthylène ;
- Les produits résultant de la réaction d'éthoxylation entre un équivalent molaire d'un acide gras, comme par exemple l'acide palmitique, l'acide myristique, l'acide laurique, l'acide stéarique, l'acide isostéarique, l'acide oléique, et entre 5 à 40 équivalents molaires d'oxyde d'éthylène ;
- Les produits résultant de la réaction d'estérification entre un acide gras, comme par exemple l'acide palmitique, l'acide myristique, l'acide laurique, l'acide stéarique, l'acide isostéarique, l'acide oléique, l'acide arachidique, l'acide béhénique et entre 4 à 20 équivalents molaires, plus particulièrement entre 3 à 10 équivalents molaires de glycérol ;

Une phase aqueuse cosmétiquement acceptable (A) comprise dans l'émulsion huile-dans-eau (E) telle que définie précédemment peut contenir classiquement un ou plusieurs solvants organiques cosmétiquement acceptables, un mélange d'eau et d'un ou plusieurs solvants organiques cosmétiquement acceptables. Les solvants cosmétiquement acceptables peuvent plus particulièrement être choisis parmi les alcools polyhydriques comme par exemple le glycérol, le diglycérol, le triglycérol, les oligomères du glycérol, le xylitol, l'érythritol, le sorbitol, le méthyl-2-propanediol-1,3; les alcools polyhydriques alcoxylés; les glycols, comme par exemple le butylène glycol, l'hexylène glycol, le caprylyl glycol ou 1,2-octanediol, le pentylène glycol ou 1,2-pentanediol, le monopropylène glycol, le dipropylène glycol, l'isoprène glycol, le butyldiglycol, les polyéthylènes glycols dont le poids moléculaire est compris entre 200g.mol-1 et 8.000g.mol-1; ou les alcools hydrosolubles comme par exemple l'éthanol, l'isopropanol ou le butanol.

L'expression « cosmétiquement acceptable » utilisée dans la définition de la phase aqueuse de l'émulsion huile-dans-eau objet de la présente invention, signifie selon la directive du Conseil de la Communauté Economique Européenne N°76/768/CEE du 27 juillet 1976 modifiée par la directive N°93/35/CEE du 14 juin 1993, que ladite phase aqueuse comprend de l'eau et toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état.

Selon un aspect particulier, l'invention a pour objet une émulsion huile-dans-eau (E) telle que définie précédemment, caractérisée en ce que ledit mélange (M₁) comprend pour 100% de sa masse :
- Une proportion massique en alcanes ramifiés supérieure ou égale à 80% et inférieure ou égale à 100%, et plus particulièrement supérieure ou égale à 90% massique ;
- Une proportion massique en alcanes linéaires supérieure ou égale à 0% et inférieure ou égale à 15%, et plus particulièrement inférieure ou égale à 10% massique,
- Une proportion massique en cyclo-alcanes supérieure ou égale à 0% et inférieure ou égale à 5%, et plus particulièrement inférieure ou égale à 1% massique,
en ce que de 95% massique à 100% massique desdits hydrocarbures cycliques ou acycliques, linéaires ou ramifiés, comportent de quinze à dix-neuf atomes de carbones et en ce que jusqu'à 5% massique desdits hydrocarbures cycliques, acycliques, linéaires ou ramifiés comportent moins de quinze atomes de carbone ou plus de dix-neuf atomes de carbone.

Selon cet aspect particulier, ledit mélange (M₁) est plus particulièrement un mélange d'hydrocarbures saturés commercialisé sous le nom de marque Emogreen^{™}L15 comprenant pour 100% de sa masse :
i) 3,7% d'alcanes linéaires comportant de quinze à dix-neuf atomes de carbone,
ii) 96% d'iso-alcanes comportant de quinze à dix-neuf atomes de carbone, et
iii) 0,3% de cyclo-alcanes comportant de quinze à dix-neuf atomes de carbone.

Selon un autre aspect plus particulier, l'émulsion à usage topique de type huile-dans-eau (E) telle définie précédemment, est caractérisée en ce que ledit mélange (M₁) comprend pour 100% de sa masse :
- Une proportion massique en alcanes ramifiés supérieure ou égale à 40% et inférieure ou égale à 100%, et plus particulièrement supérieure ou égale à 50% massique,
- Une proportion massique en alcanes linéaires supérieure ou égale à 0% et inférieure ou égale à 20%, et plus particulièrement inférieure ou égale à 15% massique,
- Une proportion massique en cyclo-alcanes supérieure ou égale à 0% et inférieure ou égale à 40% et plus particulièrement inférieure ou égale à 35% massique,
et en ce que 100% massique desdits hydrocarbures cycliques ou acycliques, linéaires ou ramifiés, comportent de quinze à dix-neuf atomes de carbones.

Selon cet aspect particulier, le mélange (M₁) est plus particulièrement un mélange d'hydrocarbures saturés commercialisé sous le nom de marque Emosmart^{™}L19, comprenant pour 100% de sa masse :
i) 13,20% massique d'alcanes linéaires comportant de 15 à 19 atomes de carbone,
ii) 55,00% massique d'iso-alcanes comportant de 15 à 19 atomes de carbone, et
iii) 31,80% de cyclo-alcanes comportant de 15 à 19 atomes de carbone.

Selon au autre aspect particulier, l'émulsion à usage topique (E) telle que définie précédemment est caractérisée en ce que la quantité efficace dudit mélange (M₁) est telle que le ratio massique entre le glycérol et ledit mélange (M₁) est inférieur ou égal à 10 et supérieur ou égal à 1, et plus particulièrement inférieur ou égal à 5 et supérieur ou égal à 2.

L'émulsion à usage topique (E) telle que définie précédemment peut comprendre un ou plusieurs adjuvants tels que :
- Des agents épaississants ou gélifiants, par exemple des polymères de type polyélectrolytes, linéaires ou branchés ou réticulés, comme l'homopolymère de l'acide acrylique partiellement ou totalement salifié, l'homopolymère de l'acide méthacrylique partiellement ou totalement salifié, l'homopolymère de l'acide 2-méthyl-[(1-oxo-2-propényl)amino]-1-propanesulfonique (AMPS) partiellement ou totalement salifié, les copolymères de l'acide acrylique et de l'AMPS, les copolymères de l'acrylamide et de l'AMPS, les copolymères de la vinylpyrolidone et de l'AMPS, les copolymères de l'AMPS et de l'acrylate de (2-hydroxyéthyle), les copolymères de l'AMPS et du méthacrylate de (2-hydroxyéthyle), les copolymères de l'AMPS et de l'hydroxyéthylacrylamide, les copolymères de l'AMPS et du N,N-diméthyl acrylamide, les copolymères de l'AMPS et du tris(hydroxy- méthyl)acrylamido méthane (THAM), les copolymères de l'acide acrylique ou méthacrylique et de l'acrylate de (2-hydroxy éthyle), les copolymères de l'acide acrylique ou méthacrylique et du méthacrylate de (2-hydroxy éthyle), les copolymères de l'acide acrylique ou méthacrylique et de l'hydroxyéthylacrylamide, les copolymères de l'acide acrylique ou méthacrylique et du THAM, les copolymères de l'acide acrylique ou méthacrylique et du N,N-diméthyl acrylamide, les terpolymères de l'acide acrylique ou méthacrylique, de l'AMPS et de l'acrylate de (2-hydroxy éthyle), les terpolymères de l'acide acrylique ou méthacrylique, de l'AMPS et du méthacrylate de (2-hydroxy éthyle), les terpolymères de l'acide acrylique ou méthacrylique, de l'AMPS et du THAM, les terpolymères de l'acide acrylique ou méthacrylique, de l'AMPS et du N,N-diméthyl acrylamide, les terpolymères de l'acide acrylique ou méthacrylique, de l'AMPS et de l'acrylamide, les copolymères de l'acide acrylique ou de l'acide méthacrylique et d'acrylates d'alkyle dont la chaîne carbonée comprend entre quatre et trente atomes de carbone et plus particulièrement entre dix et trente atomes de carbone, les copolymères de l'AMPS et d'acrylates d'alkyle dont la chaîne carbonée comprend entre quatre et trente atomes de carbone et plus particulièrement entre dix et trente atomes de carbone, les terpolymères linéaire, branché ou réticulé d'au moins un monomère possédant une fonction acide fort, libre, partiellement salifiée ou totalement salifiée, avec au moins un monomère neutre, et au moins un monomère de formule (VIII):

   CH₂=C(R'₃)-C(=O)-[CH₂-CH₂-O]ₙ-R'₄ (VIII)

   dans laquelle R'₃ représente un atome d'hydrogène ou un radical méthyle, R'₄ représente un radical alkyle linéaire ou ramifié comportant de huit à trente atomes de carbone et n représente un nombre supérieur ou égal à un et inférieur ou égal à cinquante; Les polymères de type polyélectrolytes, linéaires ou branchés ou réticulés que l'on peut associer à l'émulsion huile-dans-eau objet de la présente invention, peuvent se présenter sous la forme d'une solution, d'une suspension aqueuse, d'une émulsion eau-dans-huile, d'une émulsion huile-dans-eau, d'une poudre, par exemple les produits commercialisés sous les appellations SIMULGEL^{™} EG, SIMULGEL^{™}EPG, SEPIGEL^{™} 305, SIMULGEL^{™} 600, SIMULGEL^{™} NS, SIMULGEL^{™} INS 100, SIMULGEL^{™} FL, SIMULGEL^{™} A, SIMULGEL^{™} SMS 88, SEPINOV^{™}EMT 10, SEPIPLUS^{™}400, SEPIPLUS^{™}265, SEPIPLUS^{™}S, SEPIMAX^{™}Zen, ARISTOFLEX^{™}AVC, ARISTOFLEX^{™}AVS, NOVEMER^{™}EC-1, NOVEMER^{™}EC 2, ARISTOFLEX^{™}HMB, COSMEDIA^{™}SP, FLOCARE^{™}ET 25, FLOCARE^{™}ET 75, FLOCARE^{™}ET 26, FLOCARE^{™}ET 30, FLOCARE^{™}ET 58, FLOCARE^{™}PSD 30, VISCOLAM^{™}AT 64, VISCOLAM^{™}AT 100; les polysaccharides constitués uniquement d'oses, comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, comme les galactomannanes provenant de la gomme de cassia (DS = 1/5), de la gomme de caroube (DS = 1/4), de la gomme de tara (DS = 1/3), de la gomme de guar (DS = 1/2), de la gomme de fenugrec (DS = 1 ); les polysaccharides constitués de dérivés d'oses, comme les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme de arabique et de gomme de karaya, les glucosaminoglycanes; la cellulose, les dérivés de cellulose comme la méthyl-cellulose, l'éthyl-cellulose, l'hydroxypropyl cellulose, les silicates, l'amidon, les dérivés hydrophiles de l'amidon, les polyuréthanes;
- Des composés filmogènes,
- Des agents hydrotropes,
- Des agents plastifiants,
- Des agents opacificants et/ou nacrants, tels que les palmitate, stéarate ou les hydroxy-stéarate de sodium ou de magnésium, les monostéarates ou distéarate d'éthylène ou de polyéthylène glycol, les alcools gras, les homopolymères et copolymères de styrène tels que le styrène acrylate copolymère commercialisé sous l'appellation MONTOPOL^{™} OP1 par la société SEPPIC.
- Des agents de texture tels que la lauroyl lysine commercialisée sous l'appellation AMINOHOPE^{™}LL par la société AJINOMOTO, l'octenyl starch succinate commercialise sous l'appellation DRYFLO^{™} par la société NATIONAL STARCH, le myristyl polyglucoside commercialisé par SEPPIC sous l'appellation MONTANOV^{™} 14, les fibres de cellulose, les fibres de coton, les fibres de chitosane, le talc, la sericite, le mica ;
- Des agents surgraissants,
- Des agents séquestrants,
- Des agents chélatants,
- Des agents tensioactifs non-ioniques tels que les dérivés éthoxylés d'alcools gras comportant de 8 à 12 atomes de carbone, les dérivés éthoxylés d'acides gras comportant de 8 à 12 atomes de carbone, les dérivés éthoxylés d'esters gras comportant de 8 à 12 atomes de carbone; les dérivés éthoxylés de monoglycérides comportant de 8 à 12 atomes de carbone; les alkylpolyglycosides de formule (II):

   R₂-O-(S)_{y}-H (II),

   dans laquelle y représente un nombre décimal compris entre 1 et 5, S représente le reste d'un sucre réducteur et R₂ représente un radical alkyle linéaire ou ramifié, saturé ou insaturé, ayant de 5 à 16 atomes de carbone, de préférence de 8 à 14 atomes de carbone, ou un mélange de composés de formule (II), par exemple le caprylyl capryl glucosides, commercialisé notamment sous le nom de marque ORAMIX^{™}CG 110, le décylglucoside, commercialisé notamment sous le nom de marque ORAMIX^{™}NS 10 ;
- Des agents antioxydants, tels que l'EDTA et ses sels, l'acide citrique, l'acide tartrique, l'acide oxalique, le BHA (butylhydroxyanisol), le BHT (butylhydroxytoluène), les dérivés de tocophérol tels que l'acétate de tocophérol, des mélanges de composés antioxydants tels que la DISSOLVINE GL 47S (nom INCI: Tetrasodium Glutamate Diacetate) ;
- Des parfums,
- Des agents conservateurs,
- Des agents conditionneurs,
- Des principes actifs destinés à apporter une action traitante vis à vis de la peau ou des cheveux, tels que les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés); les composés montrant une action apaisante notamment le SEPICALM^{™} S, l'allantoïne et le bisabolol; les agents anti-inflammatoires; les composés montrant une action hydratante comme l'urée, les hydroxyurées, le glycérolglucoside, le diglycérolglucoside, les polyglycérylglucosides; les extraits végétaux riches en polyphénols comme les extraits de raisin, les extraits de pin, les extraits de vin, les extraits d'olives; les composés montrant une action amincissante ou lipolytique comme la caféine ou ses dérivés, l'ADIPOSLIM^{™}, l'ADIPOLESS^{™}, la fucoxanthine; les protéines N-acylées; les peptides N-acylés comme le MATRIXIL^{™}; les acides aminés N-acylés; les hydrolysâts partiels de protéines N-acylés; les acides aminés; les peptides; les hydrolysâts totaux de protéines; les extraits de soja, par exemple la Raffermine^{™}; les extraits de blé par exemple la TENSINE^{™} ou la GLIADINE^{™}; les extraits végétaux, tels que les extraits végétaux riches en tanins, les extraits végétaux riches en isoflavones ou les extraits végétaux riches en terpènes; les extraits d'algues d'eau douce ou marines; les extraits de plantes marines; les extraits marins en général comme les coraux; les cires essentielles; les extraits bactériens; les céramides; les phospholipides; les composés montrant une action antimicrobienne ou une action purifiante, comme le LIPACIDE^{™} C8G, le LIPACIDE^{™} UG, le SEPICONTROL^{™} A5; l'OCTOPIROX^{™} ou le SENSIVA^{™} SC50; les composés montrant une propriété énergisante ou stimulante comme le PHYSIOGENYL^{™}, le panthénol et ses dérivés comme le SEPICAP^{™} MP; les actifs anti-âge comme le SEPILIFT^{™} DPHP, le LIPACIDE^{™} PVB, le SEPIVINOL^{™}, le SEPIVITAL^{™}, le MANOLIVA^{™}, le PHYTO-AGE^{™}, le TIMECODE^{™}; le SURVICODE^{™}; les actifs anti-photo vieillissement; les actifs protecteurs de l'intégrité de la jonction dermo-épidermique; les actifs augmentant la synthèse des composants de la matrice extracellulaire comme le collagène, les élastines, les glycosaminoglycanes; les actifs agissant favorablement sur la communication cellulaire chimique comme les cytokines ou physiques comme les intégrines; les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés); les actifs améliorant la microcirculation cutanée, par exemple les veinotoniques; les actifs drainants; les actifs à visée décongestionnante comme les extraits de ginko biloba, de lierre, de marron d'inde, de bambou, de ruscus, de petit houx, de centalla asiatica, de fucus, de romarin, de saule; les agents de bronzage ou de brunissement de la peau, comme par exemple la dihydroxyacétone (DHA), l'érythrulose, l'aldéhyde mésotartrique, le glutaraldéhyde, le glycéraldéhyde, l'alloxane, la ninhydrine, les extraits végétaux comme par exemple les extraits de bois rouges du genre Pterocarpus et du genre Baphia comme le Pteropcarpus santalinus, le Pterocarpus osun, le Pterocarpus soyauxii, le Pterocarpus erinaceus, le Pterocarpus indicus ou le Baphia nitida comme ceux décrits dans la demande de brevet Européen EP 0 971 683;; les agents connus pour leur action de facilitation et/ou d'accélération du bronzage et/ou du brunissement de la peau humaine, et/ou pour leur action de coloration de la peau humaine, comme par exemple les caraténoïdes ( et plus particulièrement le beta carotène et le gamma carotène), le produit commercialisé sous le nom de marque « Carrot oil » (Nom INCI: Daucus Carota, helianthus annuus Sunflower oil) par la société Provital, qui contient des caroténoïdes, de la vitamine E et de la vitamine K; la tyrosine et/ou ses dérivés, connus pour leur effet sur l'accélération du bronzage de la peau humaine en association avec une exposition aux rayonnements ultraviolets, comme par exemple le produit commercialisé sous le nom de marque « SunTan Accelerator^{™} » par la société Provital qui contient de la tyrosine et des riboflavines (vitamine B), le complexe de tyrosine et de tyrosinase commercialisé sous le nom de marque « Zymo Tan Complex » par la société Zymo Line, le produit commercialisé sous le nom de marque MelanoBronze^{™} (nom INCI: Acetyl Tyrosine, Monk's pepper extract (Vitex Agnus-castus)) par la société Mibelle qui contient de l'acétyl tyrosine, produit commercialisé sous le nom de marque Unipertan VEG-24/242/2002 (nom INCI: butylène glycol and Acetyl Tyrosine and hydrolyzed vegetable protein and Adenosine triphosphate) par la société UNIPEX, le produit commercialisé sous le nom de marque « Try-Excell^{™} » (nom INCI: Oleoyl Tyrosine and Luffa Cylindrica (Seed) Oil and Oleic acid) par la société Sederma qui contient des extraits de pépins de courge (ou huile de Loofah), le produit commercialisé sous le nom de marque «Actibronze^{™} » (nom INCI: hydrolyzed wheat protein and acetyl tyrosine and copper gluconate) par la société Alban Muller, le produit commercialisé sous le nom de marque Tyrostan^{™} (nom INCI: potassium caproyl tyrosine) par la société Synerga, le produit commercialisé sous le nom de marque Tyrosinol (nom INCI: Sorbitan Isostearate, glyceryl oleate, caproyl Tyrosine) par la société Synerga, le produit commercialisé sous le nom de marque InstaBronze^{™} (nom INCI: Dihydroxyacetone and acetyl tyrosine and copper gluconate) commercialisé par la société Alban Muller, le produit commercialisé sous le nom de marque Tyrosilane (nom INCI: méthylsilanol and acétyl tyrosine) par la société Exymol; les peptides connus pour leur effet d'activation de la mélanogénèse comme par exemple le produit commercialisé sous le nom de marque Bronzing SF Peptide powder (nom INCI: Dextran and Octapeptide-5) par la société Infinitec Activos, le produit commercialisé sous le nom de marque Melitane (nom INCI: Glycerin and Aqua and Dextran and Acetyl hexapeptide-1) comprenant l'acétyl hexapeptide-1 connu pour son action agoniste de l'alpha-MSH, le produit commercialisé sous le nom de marque Melatimes Solutions^{™} (nom INCI: Butylene glycol, Palmitoyl Tripeptide-40) par la société LIPOTEC, les sucres et les dérivés de sucres comme par exemple le produit commercialisé sous le nom de marque Tanositol^{™} (nom INCI: inositol) par la société Provital, le produit commercialisé sous le nom de marque Thalitan^{™} (ou Phycosaccharide^{™} AG) par la société CODIF international (nom INCl: Aqua and hydrolyzed algin (Laminaria Digitata) and magnésium sulfate and manganèse sulfate) contenant un oligosaccharide d'origine marine (acide guluronique et acide mannuronique chélatés avec les ions magnésium et manganèse), le produit commercialisé sous le nom de marque Melactiva^{™} (nom INCI: Maltodextrin, Mucuna Pruriens Seed extract) par la société Alban Muller, les composés riches en flavonoïdes comme par exemple le produit commercialisé sous le nom de marque « Biotanning » (nom INCI: Hydrolyzed citrus Aurantium dulcis fruit extract) par la société Silab et connu pour être riche en flavonoides de citron (de type hespéridines);
- Des charges minérales ou des pigments, tels que le dioxyde de titane, les oxydes de fer marrons, les oxydes de fer jaune, les oxydes de fer noir, ou les oxydes de fer rouges ou encore les pigments nacrés blancs ou colorés tels que les Mica-Titane.
- Des particules procurant un effet visuel ou destinées à l'encapsulation d'actifs,
- Des particules exfoliantes,
- Des azurants optiques,
- Des agents répulsifs des insectes.

L'émulsion huile-dans-eau (E) telle que définie précédemment peut être conditionnée dans un flacon, dans un dispositif de type "flacon" pompe, sous forme pressurisées dans un dispositif aérosol, dans un dispositif muni d'une paroi ajourée comme une grille ou dans un dispositif muni d'un applicateur à billes (dit "roll-on").

L'émulsion à usage topique de type huile-dans-eau (E) telle que définie précédemment peut être utilisée en tant que produit de nettoyage et/ou de soin de la peau humaine. Ce faisant, on applique sur ladite peau humaine une quantité efficace de l'émulsion à usage topique de type huile-dans-eau (E) telle que définie précédemment. Les exemples suivants illustrent l'invention sans toutefois la limiter.

### Exemple 1 : Préparation d'émulsions huile-dans-eau selon l'utilisation revendiquée, l'émulsion comprenant un mélange (M₁), et d'émulsions huile-dans-eau comparatives.

On prépare deux émulsions huile-dans-eau selon l'utilisation revendiquée, notées (E₁) et (E₂), dont les proportions massiques de leurs constituants sont indiquées dans le tableau 1, et 8 émulsions huile-dans-eau comparatives notées (F₁) à (F₆) dont les proportions massiques de leurs constituants sont indiquées dans le tableau 2 ci-dessous. Le procédé de préparation commun pour les émulsions huile-dans-eau comprenant un mélange (M₁) J 2. et pour les émulsions huile-dans-eau comparatives, est le suivant :
- On verse dans un bécher, à une température de 75 °C, le mélange (M₁) testé, puis on disperse progressivement et selon les cas successivement le caprylic/capric triglycéride, le Simulsol^{™}165, l'hexadécanol-1, l'acide stéarique, le Sepiplus^{™}S (Polyélectrolyte épaississant), le Primol^{™}352, le Sepicide^{™}HB sous agitation mécanique à 80 tours/minute ;
- On verse dans un bécher, à une température de 75°C, la phase aqueuse comprenant l'eau, le glycérol et l'EDTA tétrasodique,
- Le contenu du bécher comprenant la phase grasse, est progressivement ajouté à la phase aqueuse à une température de 75°C, puis placé sous l'action d'un système de rotor-stator pendant 4 minutes à une vitesse de 4 000 tours par minute
- Le mélange alors obtenu, est refroidi sous agitation de type ancre pendant vingt minutes, puis vidangé pour obtenir les émulsions huile-dans-eau (E₁) à (E₂) selon l'invention et les émulsions huile-dans-eau comparatives (F₁) à (F₆).

**Tableau 1**

| | **Emulsion** | | |
|---|---|---|---|
| | **(E₁)** | **(E₂)** | **(E₃)** |
| **Phase grasse** | | | |
| Triglycérides C₈-C¹⁰ | 3% | 3% | 4% |
| Sepiplus^{™} S⁽¹⁾ | 1% | 1% | 1% |
| Simulsol^{™} 165⁽²⁾ | 2% | 2% | 2% |
| Hexadécanol-1 | 1,5% | 1,5% | 2,5% |
| Primol 352⁽³⁾ | 4% | 4% | 5% |
| Acide stéarique | 0,2% | 0,2% | 0,2% |
| Sepicide HB⁽⁴⁾ | 1% | 1% | 1% |
| Emosmart^{™}L19⁽⁵⁾ | 5% | 0% | 0% |
| Emogreen^{™}L15⁽⁶⁾ | 0% | 5% | 5% |

| **Phase aqueuse** | | | |
|---|---|---|---|
| Eau | Qs 100% | Qs 1 00% | Qs 100% |
| Glycérol | 10% | 10% | 10% |
| EDTA tétrasodique | 0,2% | 0,2% | 0,2% |

**Tableau 2**

| | **Emulsion** | | |
|---|---|---|---|
| | **(F₁)** | **(F₂)** | **(F₃)** |
| **Phase grasse** | | | |
| Triglycérides C₈-C₁₀ | 3% | 3% | 3% |
| Sepiplus^{™} S⁽¹⁾ | 1% | 1% | 1% |
| Simulsol^{™} 165⁽²⁾ | 2% | 2% | 2% |
| Hexadécanol-1 | 1,5% | 1,5% | 1,5% |
| Primol 352⁽³⁾ | 4% | 4% | 4% |
| Acide stéarique | 0,2% | 0,2% | 0,2% |
| Sepicide HB⁽⁴⁾ | 1% | 1% | 1% |
| Emosmart^{™}L15⁽⁷⁾ | 5% | 0% | 0% |
| Emosmart^{™} V21⁽⁸⁾ | 0% | 5% | 0% |
| Isohexadécane | 0% | 0% | 5% |

| **Phase aqueuse** | | | |
|---|---|---|---|
| Eau | Qs 100% | Qs 100% | Qs 100% |
| Glycérol | 10% | 10% | 10% |
| EDTA tétrasodique | 0,2% | 0,2% | 0,2% |

**Tableau 2 (suite)**

| | **Emulsion** | | | | |
|---|---|---|---|---|---|
| | **(F₄)** | **(F₅)** | **(F₆)** | **(F₇)** | **(F₈)** |
| **Phase grasse** | | | | | |
| Triglycérides C₈-C₁₀ | 3% | 3% | 3% | 4% | 4% |
| Sepiplus^{™} S⁽¹⁾ | 1% | 1% | 1% | 1% | 1% |
| Simulsol^{™} 165⁽²⁾ | 2% | 2% | 2% | 2% | 2% |
| Hexadécanol-1 | 1,5% | 1,5% | 1,5% | 2,5% | 2,5% |
| Primol 352⁽³⁾ | 4% | 4% | 4% | 5% | 5% |
| Acide stéarique | 0,2% | 0,2% | 0,2% | 0,2% | 0,2% |
| Sepicide HB⁽⁴⁾ | 1% | 1% | 1% | 1% | 1% |
| Isododécane | 5% | | | | |
| Lanol^{™} 99⁽⁹⁾ | | 5% | | | |
| DC 345⁽¹⁰⁾ | | | 5% | 5% | 5% |

| **Phase aqueuse** | | | | | |
|---|---|---|---|---|---|
| Eau | Qs 100% | Qs 100% | | Qs 100% | Qs 100% |
| Glycérol | 10% | 10% | 10% | 10% | 20% |
| EDTA tétrasodique | 0,2% | 0,2% | 0,2% | 0,2% | 0,2% |

| | | | | | |
|---|---|---|---|---|---|
| (1) (Sepiplus^{™}S): Agent épaississant (nom INCI: hydroxy ethyl acrylate/sodium acryloyldimethyl taurate copolymer & Polyisobutene & PEG-7 trimethylolpropane coconut oil); (2) (Simulsol^{™} 165): Agent émulsionnant (nom INCI, PEG-100 stearate & Glyceryl stearate); (3) (Primol^{™}352): Agent émollient (nom INCI Huile de paraffine); (4) (Sepicide^{™}HB): Agent conservateur (nom INCI: phenoxyethanol / methylparaben / ethylparaben / propylparaben / isobutylparaben) ; (5) (Emosmart^{™}L19): Mélange d'hydrocarbures saturés cycliques ou acycliques, linéaires ou ramifiés comprenant pour 100% de sa masse: i) 13,20 % massique d'alcanes linéaires comportant de 15 à 19 atomes de carbone, ii) 55,00% massique d'iso-alcanes comportant de 15 à 19 atomes de carbone, iii) 31,80 % de cyclo-alcanes comportant de 15 à 19 atomes de carbone; (6) (Emogreen^{™}L15): Mélange comprenant pour 100% de sa masse: i) 3,7% d'alcanes linéaires comportant de 15 à 19 atomes de carbone, ii) 96% % d'iso-alcanes comportant de 15 à 19 atomes de carbone, iii) 0,3 % de cyclo-alcanes comportant de 15 à 19 atomes de carbone; (7) (Emosmart^{™}L15): Mélange comprenant pour 100% de sa masse: i) 9,26% massique d'alcanes linéaires comportant de 13 à 15 atomes de carbone, ii) 39,06 % massique d'iso-alcanes comportant de 13 à 15 atomes de carbone, iii) 51,68 % de cyclo-alcanes comportant de 13 à 15 atomes de carbone ; (8) (Emosmart^{™}V21): Mélange comprenant pour 100% de sa masse: i) 15,99 % massique d'alcanes linéaires comportant de 18 à 21 atomes de carbone, ii) 59,90% massique d'iso-alcanes comportant de 18 à 21 atomes de carbone, iii) 24,11 % de cyclo-alcanes comportant de 18 à 21 atomes de carbone, (9) (Lanol^{™} 99): Agent émollient (nom INCI isononyl isononate); (10) (DC 345): (nom INCI: clyclopentasiloxane & cyclohexasiloxane. | | | | | |

### Evaluation des propriétés collantes des émulsions huile-dans-eau,

### comprenant un mélange (M₁), et d'émulsions huile-dans-eau comparatives.

### Principe de la mesure

Il s'agit de caractériser chaque émulsion testée par une valeur moyenne, de 3 valeurs mesurées de la force d'adhésion que subit le plan Peltier lors des compressions et remontées.

### Matériel et appareillage

Les mesures sont réalisées AU MOYEN d'un rhéomètre de modèle DHR2 (de marque TA Instrument) équipé d'un support de type plan Peltier sur lequel est posé une surface de plexiglas sur laquelle est déposée l'émulsion à tester.

### Mode opératoire

On dépose une quantité de 63 microlitres de l'émulsion à caractériser sur une plaque de plexiglas, disposée sur un plan Peltier, dont la température est régulée à 40°C. Après dépôt de l'échantillon, on étale par une contrainte rotationnelle du plan mobile supérieur sur chaque émulsion, pendant 10 secondes. On mesure la force normale qui retient le plan mobile supérieur au plan Peltier (ou force d'adhésion) lors d'une succession de 48 mouvements de compression suivi de remontées.

### Expression des résultats

Pour chaque émulsion testée, on considère les valeurs obtenues entre 4000 et 6000s du cycle de compression/tension. La moyenne et les écart-types sont calculés. Le test est répété au moins 3 fois, la valeur moyenne globale de la tension ou force d'adhésion est alors déterminée.

### Résultats

Les résultats obtenus sont consignés dans le tableau 3 ci-après.

**Tableau 3**

| **Emulsion testée** | **(E₁)** | **(E₂)** | **(E₃)** | **(F₁)** | **(F₂)** |
|---|---|---|---|---|---|
| Moyenne de la force d'adhésion (en Newton) | 2,28 | 5,06 | 4,78 | 11,9 | 6,23 |

**Tableau 3 (suite)**

| **Emulsion testée** | **(F₃)** | **(F₄)** | **(F₅)** | **(F₆)** | **(F₇)** | **(F₈)** |
|---|---|---|---|---|---|---|
| Moyenne de la force d'adhésion (en Newton) | 8,21 | 11,42 | 3,76 | 7,67 | 7,83 | 11,57 |

### Analyse des Résultats

Les résultats consignés dans le tableau 3 font apparaître clairement que les moyennes des forces mesurées pour les émulsions (E₁) (E₂) et (E₃) sont inférieures aux moyennes enregistrées pour les émulsions comparatives (F₁) à (F₈), et plus particulièrement inférieures aux valeurs de 7,67 liées respectivement aux émulsions de référence (F₆), (F₇) et (F₈) comprenant l'agent de référence DC345.

## Revendications

1. Utilisation d'une quantité efficace d'un mélange (M₁) d'hydrocarbures saturés cycliques ou acycliques, linéaires ou ramifiés parmi lesquels au moins 95% massique comportent de quinze à dix-neuf atomes de carbone pour améliorer les propriété sensorielles d'une émulsion à usage topique (E₀) de type-huile-dans-eau comprenant pour 100% de sa masse, de 1% à 30% massique de glycérol, dans le but de réduire ou d'annihiler l'effet collant induit par la présence de glycérine sans qu'il soit nécessaire d'épaissir la phase aqueuse avec des polymères synthétiques comportant des motifs siliconés ou fluorés, ni d'ajouter un tiers composé de nature siliconée.

2. Utilisation telle que définie à la revendication 1, **caractérisée en ce que** ledit mélange (M₁) comprend pour 100% de sa masse :
- Une proportion massique en alcanes ramifiés supérieure ou égale à 80 % et inférieure ou égale à 100%,
- Une proportion massique en alcanes linéaires supérieure ou égale à 0% et inférieure ou égale à 15%,
- Une proportion massique en cyclo-alcanes supérieure ou égale à 0% et inférieure ou égale à 5%,
**en ce que** de 95% massique à 100% massique desdits hydrocarbures cycliques ou acycliques, linéaires ou ramifiés, comportent de quinze à dix-neuf atomes de carbones **et en ce que** jusqu'à 5% massique desdits hydrocarbures cycliques, acycliques, linéaires ou ramifiés comportent moins de quinze atomes de carbone ou plus de dix-neuf atomes de carbone.

3. Utilisation telle que définie à la revendication 1, **caractérisée en ce que** ledit mélange (M₁) comprend pour 100% de sa masse :
- Une proportion massique en alcanes ramifiés supérieure ou égale à 40% et inférieure ou égale à 100%,
- Une proportion massique en alcanes linéaires supérieure ou égale à 0% et inférieure ou égale à 20%,
- Une proportion massique en cyclo-alcanes supérieure ou égale à 0% et ou égale à 40%, **et en ce que** 100% massique desdits hydrocarbures cycliques ou acycliques, linéaires ou ramifiés, comportent de quinze à dix-neuf atomes de carbone.

4. Utilisation telle que définie à l'une quelconque des revendications 1 à 3 **caractérisée en ce que** la quantité efficace dudit mélange (M₁) est telle que le ratio massique entre le glycérol et ledit mélange (M₁) est inférieur ou égal à 10 et supérieur ou égal à 1, et plus particulièrement inférieur ou égal à 5 et supérieur ou égal à 2.

## Patentansprüche

1. Verwendung einer wirksamen Menge einer Mischung (M₁) von cyclischen oder acyclischen, linearen oder verzweigten gesattigten Kohlenwasserstoffen, von denen mindestens 95 Massen-% fünfzehn bis neunzehn Kohlenstoffatomeenthalten, zur Verbesserung der sensorischen Eigenschaften einer Emulsion für die topische Verwendung (E₀) vom Öl-in-Wasser-Typ, die auf 100 % ihrer Masse, 1 bis 30 Massen-% Glycerin enthält, mit dem Ziel, den durch die Anwesenheit von Glycerin induzierten Klebrigkeitseffekt zu reduzieren oder zu beseitigen, ohne dass es erforderlich ist, die wässrige Phase mit synthetischen Polymeren zu verdicken, die Silikon- oder Fluoreinheiten umfassen, oder eine silikonhaltige Drittverbindung hinzuzufügen.

2. Verwendung nach Anspruch 1, **dadurch gekennzeichnet, dass** die Mischung (M₁) auf 100 % ihrer Masse Folgendes enthält:
- einen Massenanteil an verzweigten Alkanen größer oder gleich 80 % und kleiner oder gleich 100 %,
- einen Massenanteil an linearen Alkanen größer oder gleich 0 % und kleiner oder gleich 15 %,
- einen Massenanteil an Cycloalkanen größer oder gleich 0 % und kleiner oder gleich 5 %,
**dass** 95 Massen-% bis 100 Massen-% der cyclischen oder acyclischen, linearen oder verzweigten Kohlenwasserstoffe fünfzehn bis neunzehn Kohlenstoffatome enthalten **und dass** bis zu 5 Massen-% der cyclischen, acyclischen, linearen oder verzweigten Kohlenwasserstoffe weniger als fünfzehn Kohlenstoffatome oder mehr als neunzehn Kohlenstoffatome enthalten.

3. Verwendung nach Anspruch 1, **dadurch gekennzeichnet, dass** die Mischung (M₁) auf 100 % ihrer Masse Folgendes enthält:
- einen Massenanteil an verzweigten Alkanen größer oder gleich 40 % und kleiner oder gleich 100 %,
- einen Massenanteil an linearen Alkanen größer oder gleich 0 % und kleiner oder gleich 20 %,
- einen Massenanteil an Cycloalkanen größer oder gleich 0 % und kleiner oder gleich 40 %,
**und dass** 100 Massen-% der cyclischen oder acyclischen, linearen oder verzweigten Kohlenwasserstoffe fünfzehn bis neunzehn Kohlenstoffatome enthalten.

4. Verwendung nach einem der Ansprüche 1 bis 3, **dadurch gekennzeichnet, dass** die wirksame Menge der Mischung (M₁) so beschaffen ist, dass das Massenverhältnis zwischen dem Glycerin und der Mischung (M₁) kleiner oder gleich 10 und größer oder gleich 1 und spezieller kleiner oder gleich 5 und größer oder gleich 2 ist.

## Claims

1. Use of an effective amount of a mixture (M₁) of cyclic or acyclic, linear or branched saturated hydrocarbons, among which at least 95% by weight comprise from fifteen to nineteen carbon atoms, for improving the sensory properties of an oil-in-water type emulsion for topical use (E₀) comprising, for 100% of its weight, from 1% to 30% by weight of glycerol, with the aim of reducing or dispensing with the tacky effect induced by the presence of glycerol without it being necessary to thicken the aqueous phase with synthetic polymers comprising silicone or fluoro units or to add a third compound of silicone nature.

2. Use as defined in Claim 1, **characterized in that** said mixture (M₁) comprises, for 100% of its weight:
- a weight proportion of branched alkanes of greater than or equal to 80% and less than or equal to 100%,
- a weight proportion of linear alkanes of greater than or equal to 0% and less than or equal to 15%,
- a weight proportion of cycloalkanes of greater than or equal to 0% and less than or equal to 5%,
**in that** from 95% by weight to 100% by weight of said cyclic or acyclic, linear or branched hydrocarbons comprise from fifteen to nineteen carbon atoms **and in that** up to 5% by weight of said cyclic, acyclic, linear or branched hydrocarbons comprise less than fifteen carbon atoms or more than nineteen carbon atoms .

3. Use as defined in Claim 1, **characterized in that** said mixture (M₁) comprises, for 100% of its weight:
- a weight proportion of branched alkanes of greater than or equal to 40% and less than or equal to 100%,
- a weight proportion of linear alkanes of greater than or equal to 0% and less than or equal to 20%,
- a weight proportion of cycloalkanes of greater than or equal to 0% and less than or equal to 40%,
**and in that** 100% by weight of said cyclic or acyclic, linear or branched hydrocarbons comprise from fifteen to nineteen carbon atoms.

4. Use as defined in any one of Claims 1 to 3 **characterized in that** the effective amount of said mixture (M₁) is such that the weight ratio between the glycerol and said mixture (M₁) is less than or equal to 10 and greater than or equal to 1, and more particularly less than or equal to 5 and greater than or equal to 2.
